# EUROPEAN PATENT SPECIFICATION

(11) **EP 1 560 558 B1**
(45) Date of publication and mention of the grant of the patent: **29.12.2010**
(21) Application number: 03759035.3
(22) Date of filing: 30.10.2003
(51) Int. Cl.: A61Q 7/00, A61K 8/64, A61K 38/13, C07K 7/64

(54) **USE OF 3-POSITION CYCLOSPORIN DERIVATIVES FOR HAIR GROWTH**
VERWENDUNG VON 3-POSITIONS-CYCLOSPORIN-DERIVATEN FÜR HAARWACHSTUM
UTILISATION DE DERIVES DE CYCLOSPORINE EN POSITION 3 POUR LA CROISSANCE CAPILLAIRE

(30) Priority: 04.11.2002 KR 2002067751
(43) Date of publication of application: 10.08.2005
(73) Proprietor: LG Household & Health Care Ltd., Seoul 150-010 (KR)
(72) Inventor: KIM, Sang-Nyun, Daejeon 305-728 (KR); YOON, Yeo-Kyeong, Seoul 151-762 (KR); KIM, Moon-Moo, 301-401, Mangmi 2-dong, Suyeong-gu, Busan 613-774 (KR); KIM, Jong-Il, Daejeon 305-728 (KR); KIM, Seung-Jin, Daejeon 305-340 (KR); KIM, Hyung-Jin, Daejeon 305-340 (KR); LEE, Heon-Sik, Daejeon 305-340 (KR)
(74) Representative: Schrell, Andreas
(86) International application number: PCT/KR2003/002305
(87) International publication number: WO 2004/041221

(56) References cited:
- WO-A-01/35914
- WO-A1-01/35913
- WO-A1-99/65933
- KR-A- 20010 025 682
- US-A- 4 703 033
- US-A- 4 771 122
- US-A- 5 965 527
- US-A1- 2001 025 025

## Description

### Technical Field

The present invention relates to a hair growth promoting agent comprising a cyclosporin derivative as an active ingredient and more particularly, to a hair growth promoting agent comprising cyclosporin derivatives modified in the 3-position as an active ingredient.

### Background Art

On average, the human scalp contains about 100,000 to 150,000 hairs. Each hair has three main stages of growth: anagen, catagen and telogen, after which the hair falls out. This hair growth cycle is repetitive and the duration of one cycle is different from other cycles, ranging approximately 3 to 6 years. Thus, the average adult normally loses about 50 to 100 hairs every day. In general, alopecia refers to a phenomenon wherein duration of the anagen growth phase is shortened and the percentage of hairs in the catagen and telogen phases increases, whereby the number of lost hairs is increased excessively and abnormally.

There are many theories to explain for loss of hair, including for example, poor blood circulation, excessive functioning of male sex hormone, excessive production and secretion of sebum, deterioration of scalp by peroxides, bacteria, etc., hereditary factors, aging, stress, etc. However, explicit mechanisms have not been revealed. Recently, the population suffering from hair loss is tending to increase, since changing dietary habits and stress imposed on individuals due to modem social environments, etc. has increased. Also, the age of the individuals affected by alopecia is dropping and furthermore, the population of female alopecia sufferers is rising.

One of preparations which are most commonly used for treatment and prevention of alopecia is one that contains minoxidil. There are two hair-regrowth agents which have received approval from the U.S. Food and Drug Administration, and minoxidil is one of those approved hair-regrowth agents. Minoxidil was originally developed as a hypertension drug for the purpose of reducing blood pressure. However, when using this drug, as a side effect, a trichogenous effect was observed and thereafter, this drug became famous as a hair-regrowth agent. Although mechanisms by which minoxidil works as a hair-regrowth agent is not clearly understood, it is inferred that minoxidil increases blood flow by expansion of blood vessels, whereby roots of hairs are supplied with more nutrition and eventually, growth of hairs are promoted.

Such a model of blood flow increase has been indirectly supported by a recent report that minoxidil enhances the expression of vascular endothelial growth factor (VEGF), a growth factor associated with vasodilatation in the dermal papilla which is a main cell making up the hair roots. Also, other than the vasodilative effect of the minoxidil in the hair-restoring mechanism, it has been reported that minoxidil promotes activation of dermal papilla cells in the roots of hair incubated *in vitro,* and growth of hair follicles in a tissue culture of follicles *in vitro.* These facts indicate that minoxidil may work directly on the roots of hair as a growth factor.

In addition, finasteride, a main component of Propecia which has started to be sold by Merck, is used for treatment of alopecia. It inhibits conversion of the male hormone testosterone into dihydrotestosterone, which is a more potent male hormone than testosterone. On December of 1997, the 1 mg finasteride tablet was approved by the US FDA as a hair-regrowth agent for treatment of male pattern hair loss in men only, and is now commercially available. In clinical studies, it has been demonstrated to have a significant trichogenous effect. However, there has been a report that finasteride may inhibit male sexual function as a side effect. Since neither finasteride nor minoxidil show superior effect in clinical tests, and there is concern about side effects, many researches are conducted to develop a new and improved hair-regrowth agents.

The cyclosporin family of drugs has immunosuppressive activity. It is also effective to inhibit growth of virus, fungus, protozoan, etc. and has various physiological effects such as nephrotoxicity, hepatotoxicity; hypertension, enlargement of periodontium, trichogenous effect, and so on, as side effects. Cyclosporin A, a representative cyclosporin, is a cyclic peptide having the following Chemical Formula, which comprises 11 amino acids, including several N-methyl amino acids and D-alanine at No. 8 residue. 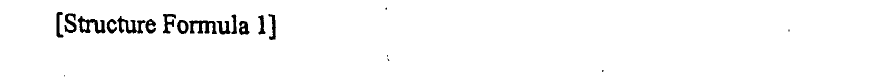 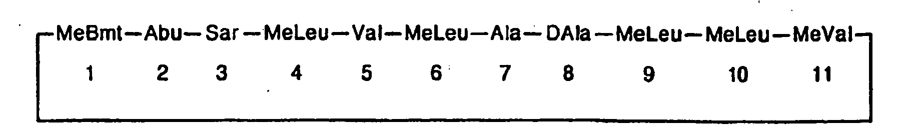 where MeBmt is N-methyl-(4R)-4-[(E)-2-butenyl]-4-methyl-L-threonine, Abu is L-α-aminobutyric acid, Sar is sarcosine, MeLeu is N-methyl-L-leucine,Val is L-valine, Ala is L-alanine, DAla is D-alanine, and MeVal is N-methyl-L-valine.

The amino acid form of cyclosporin A of the above Chemical Formula 1 is L-configuration, unless otherwise specified. The residue numbering of amino acids starts from MeBmt and proceeds clockwise, i.e. 1 for MeBmt and 11 for the last MeVal (N-methyl-L-valine) as shown in the Structure Formula 1. Nomenclature of various derivatives including cyclosporins A to Z, follows methods commonly used. For example, if Abu in the 2-position of cyclosporin A is substituted with L-alanine, L-threonine, L-valine or L-norvaline, the derivatives thus prepared are named cyclosporin B, cyclosporin C, cyclosporin D or cyclosporin G, respectively. Further, when the amino acid residues of the cyclosporin derivatives differ from those of cyclosporin A, the derivatives are named by describing the substituent. For example, if sarcosine, being the amino acid residue 3 of cyclosporin A, is substituted with [D-2-ethylthio-sar³] or [D-2-propylthio-sar³], the derivatives thus prepared are named [D-2-ethylthio-sar³] cyclosporin A or [D-2-propylthio-sar³] cyclosporin A, respectively. Meanwhile, a common method for abbreviating amino acids is employed, that is, N-methyl-L-leucine is abbreviated by MeLeu, N-methyl-L-isoleucine by MeIle, N-methyl-L-Valine by MeVal, N-methyl-L-alanine by MeAla, N-methyl-L-norvaline by MeNva, L-leucine by Leu, L-isoleucine by Ile, sarcosine by Sar, L-serine by Ser, L-valine, Val, L-alanine by Ala, D-alanine by DAla, L-aminobutyric acid by Abu, L-threonine by Thr, and L-norvaline by Nva.

So far, possible development of cyclosporin as a hair-regrowth agent has been studied by many research groups. Particularly, researches involving animal hair regrowth tests, human alopecia areata, human male pattern alopecia, and inhibition effect of hair loss by chemotherapy in animal models have been widely conducted. In comparative experiments on mouse's back, it is shown that cyclosporin has a hair regrowth effect about 100 times superior to minoxidil Based on such findings, there have been attempts to utilize cyclosporin as a treatment for male pattern alopecia, and many applications for patents have been filed.

For example, Japanese Patent Publication Kokai Nos. Sho 60-243008, Sho 62-19512 and Sho 62-19513 disclose use of cyclosporin derivatives as a hair regrowth agent. Also, Europe Patent Publication No. 0414b32B1 teaches a cyclosporin derivative modified in the 8-position, and PCT Publication No. 93/17039 teaches isocyclosporin. Moreover, United States Patent No. 5,807,820 and British Patent No. 2,218,334A disclose cyclosporins with excellent transdermal absorption, pursuant to the use of cyclosporins as hair restorers.

WO 01/359 14 A1 and WO 01/359 13 A1 disclose the use of cyclosporin A, B, C, D and G derivatives, containing a hydroxyl group in the side chain of the amino acid in 4-position, as a hair growth promoting agent.

### Disclosure of the Invention

Therefore, the present invention has been made in view of the above problems associated with side effects of cyclosporin A, and it is an object of the present invention to provide a hair growth promoting agent comprising a cyclosporin derivative as an active ingredient, which exerts an excellent hair growth-promotion ability. With the aim of developing a novel agent with hair growth promoting effect, the present inventors chemically synthesized a variety of 3-position analogs of cyclosporin, and hair growth promoting effects thereof were examined, Thus, the invention provides a hair growth promoting agent comprising a cyclosporin derivative as an active ingredient,

In accordance with one aspect of the present invention, the above and other objects can be accomplished by the provision of a hair growth promoting agent comprising a 3-position analog of cyclosporin represented by the below Formula 1, as an active ingredient, which is prepared by synthesizing a variety of derivatives thereof and evaluating their hair growth promoting effects, with an aim of developing a novel agent for promoting hair growth. 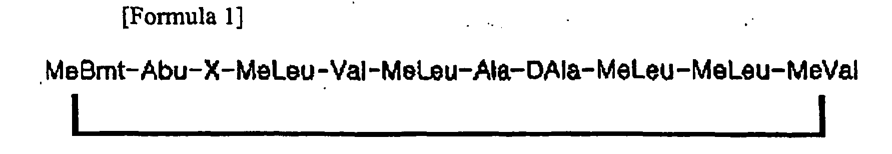 wherein:
MeBmt represents N-methyl-(4R)-4-[(E)-butenyl]-4-methyl-L-threonine;
Abu represents L-aminobutyric acid;
X represents [D-2-ethylthio-sarcosine], [D-2-propylthio-sarcosine], [D-2-isopropylthio-sarcosine], [D-2-allylthio-sarcosine], [D-2-benzylthio-sarcosine], [D-2-(4-nitrophenyl)thio-sarcosine] or [D-2-(dimethylthiocarbamyl)dithio-sarcosine];
MeLeu represents N-methyl-L-leucine;
Val represents L-valine;
Ala represents L-alanine;
DAla represents D-alanine;
MeVal represents N-methyl-L-valine.

In accordance with yet another aspect of the present invention, there is provided a hair growth promoting agent, whose composition comprising a 3-position analog of cyclosporin may be formulated in the form of liquid formulations, sprays, gels, pastes, emulsions, creams, conditioners or shampoos.

### Brief Description of the Drawings

The above and other objects, features and other advantages of the present invention will be more clearly understood from the following detailed description taken in conjunction with the accompanying drawing, in which:
Fig. 1 is a ¹H-NMR spectrum of [D-2-ethylthio-sar³] cyclosporin A;
Fig. 2 is a ¹³C-NMR spectrum of [D-2-ethylthio-sar³] cyclosporin A;
Fig. 3 is a ¹H-NMR spectrum of [D-2-propylthio-sar³] cyclosporin A;
Fig. 4 is a ¹³C-NMR spectrum of [D-2-propylthio-sar³] cyclosporin A;
Fig. 5 is a ¹H-NMR spectrum of [D-2-isopropylthio-sar³] cyclosporin A;
Fig. 6 is a ¹³C-NMR spectrum of [D-2-isopropylthio-sar³] cyclosporin A;
Fig. 7 is a ¹H-NMR spectrum of [D-2-allylthio-sar³] cyclosporin A;
Fig. 8 is a ¹³C-NMR spectrum of [D-2-allylthio-sar³] cyclosporin A;
Fig. 9 is a ¹H-NMR spectrum of [D-2-benzylthio-sar³] cyclosporin A;
Fig. 10 is a ¹³C-NMR spectrum of [D-2-benzylthio-sar³] cyclosporin A;
Fig. 11 is a ¹H-NMR spectrum of [D-2-(4-nitrophenyl)thio-sar³] cyclosporin A;
Fig. 12 is a ¹³C-NMR spectrum of [D-2-(4-nitrophenyl)thio-sar³] cyclosporin A;
Fig. 13 is a ¹H-NMR spectrum of [D-2-(dimethylthiocarbamyl)thio-sar³] cyclosporin A; and
Fig. 14 is a ¹³C-NMR spectrum of [D-2-(dimethylthiocarbamyl)thio-sar³] cyclosporin A.

### Best Mode for Carrying Out the Invention

Hereinafter, the present invention will be described in detail, in conjunction with various examples. These examples are provided only for illustrative purposes, and the present invention is not to be construed as being limited to those examples.

### Example 1: Synthesis of 3-position analog of cyclosporin

A general method for the alkylation of cyclosporin A was as follows. Tetrahydrofuran (THF) was added with diisopropyl amine ((i-Pr)₂NH) and added with a solution of n-butyl lithium (BuLi) in hexane under nitrogen atmosphere at -78 °C, followed by stirring for 30 min. To the solution of LDA (lithium diisopropylamide) thus prepared, cyclosporin A in THF was added, stirred for 1 hr, and electrophile was added.

### 1-1: Synthesis of [D-2-ethylthio-sar³]cyclosporin A: Compound 1

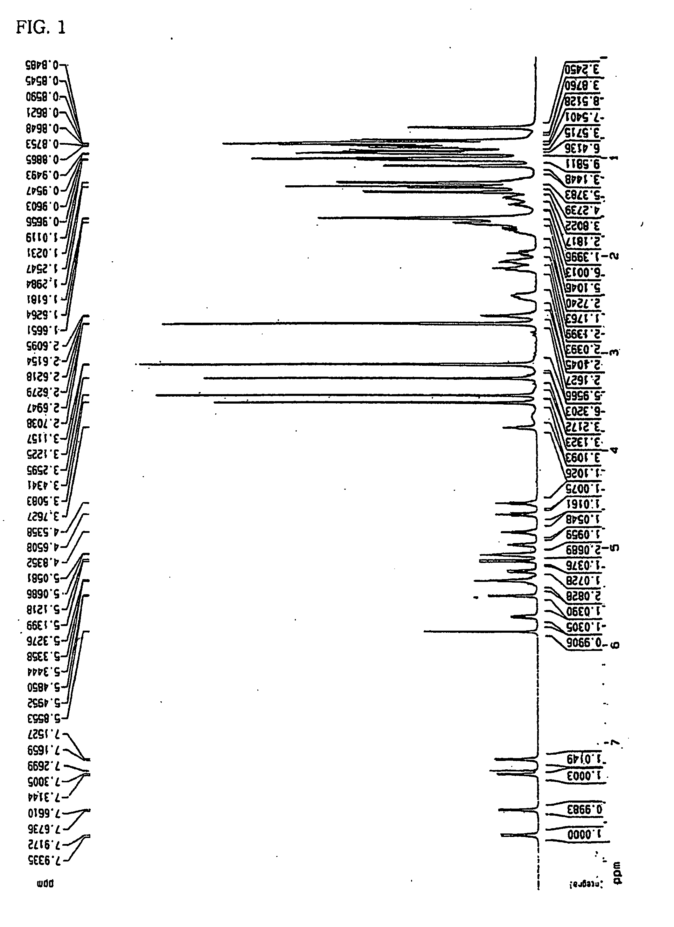

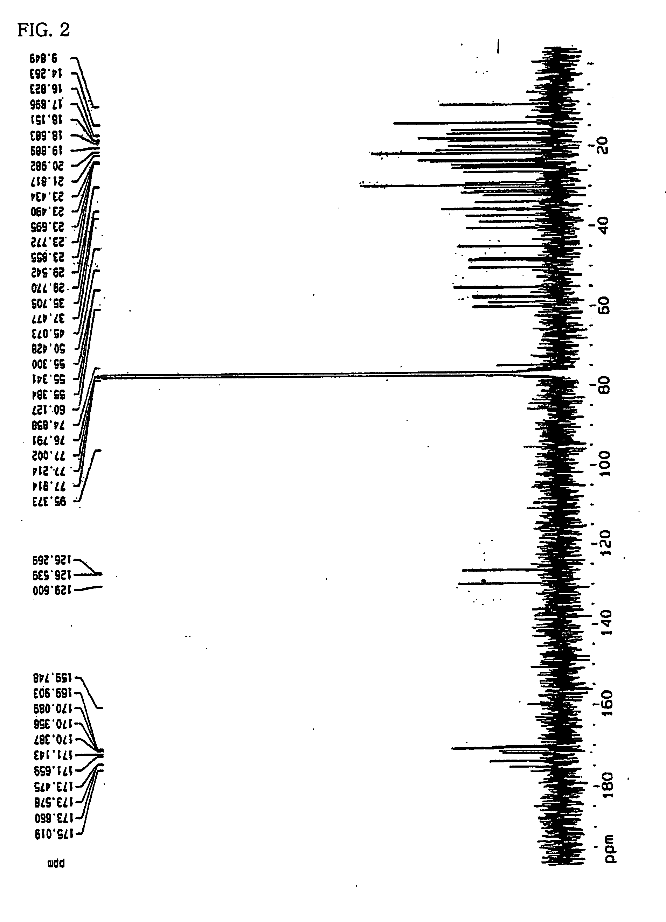

According to the general method above, THF (100 ml), (i-Pr)₂NH (1.6 ml), BuLi (4.0 ml), cyclosporin A (1.0 g) dissolved in THF (10 ml) and ethyl disulfide (Et₂S₂, 1.6 ml) were used. The reaction mixture was stirred for 24 hrs at 0 °C and added with 20 ml water, followed by concentration. The residue was added with ethyl acetate (EtOAc), washed with water and a solution of saturated sodium chloride in sequence, and dried over anhydrous MgSO₄. After concentrating, the residue was subjected to silica gel column chromatography (100 g silica gel, dichloromethane : methylalcohol = 100:1 ~ 50:1), followed by HPLC to give 0.32 g of the title compound. Molecular weight of the compound was determined by FAB MS (ZMS AX 505H) analysis. To confirm the molecular structure, Nuclear Magnetic Resonance (NMR) measurements were performed on 600 MHz (Bruker) for ¹H-NMR and on 150 MHz (Bruker) for ¹³C-NMR, and the spectra are shown in Figs. 1 and 2, respectively.

### 1-2: Synthesis of [D-2-propylthio-sar³] cyclosporin A: Compound 2

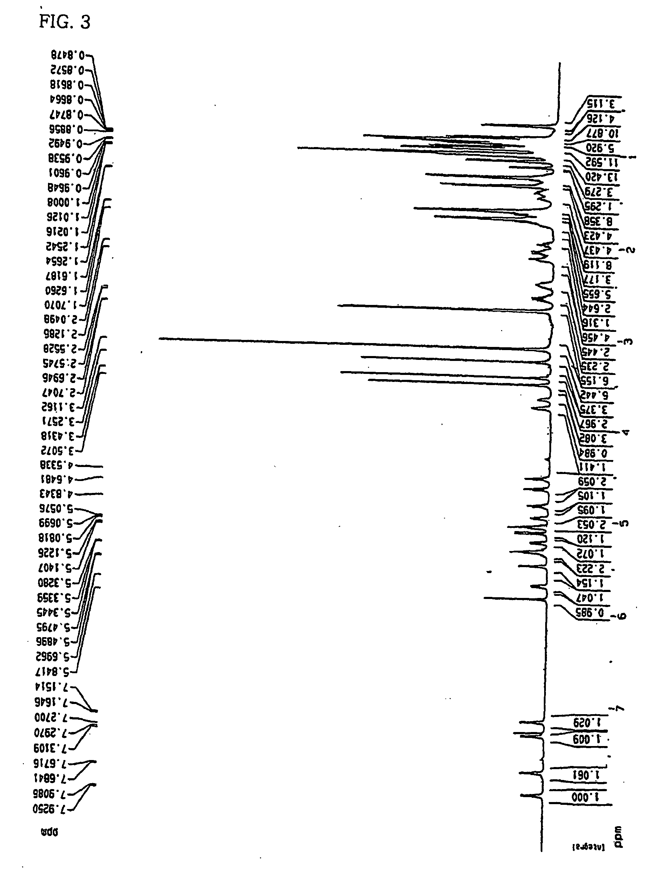

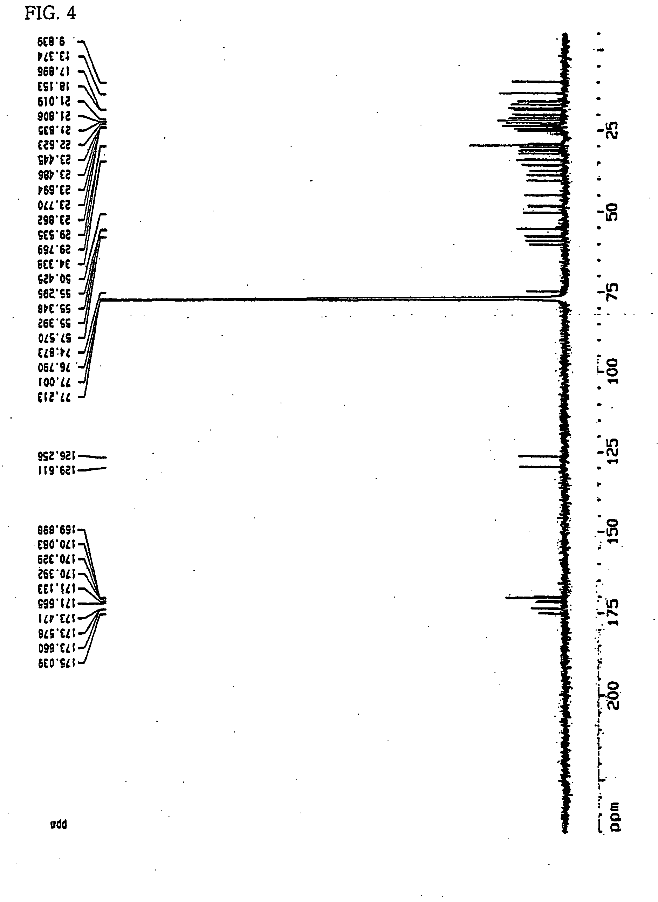

According to the general method above, THF (100 ml), (i-Pr)₂NH (1.6 ml), BuLi (4.0 ml), cyclosporin A (1.0 g) dissolved in THF (10 ml) and propyl disulfide (Pr₂S₂, 1.8 ml) were used. The reaction mixture was stirred for 24 hrs at 0 °C and added with 20 ml water, followed by concentration. The residue was added with ethyl acetate (EtOAc), washed with water and a solution of saturated sodium chloride in sequence, and dried over anhydrous MgSO₄. After concentrating, the residue was subjected to silica gel column chromatography (100 g silica gel, dichloromethane : methylalcohol = 100:1 ~ 50:1), followed by HPLC to give 0.21 g of the title compound. Molecular weight of the compound was determined by FAB MS (ZMS AX 505H) analysis. To confirm the molecular structure, Nuclear Magnetic Resonance (NMR) measurements were performed on 600 MHz (Bruker) for ¹H-NMR and on 150 MHz (Bruker) for ¹³C-NQ4R, and the spectra are shown in Figs. 3 and 4, respectively.

### 1-3: Synthesis [D-2-isopropylthio-sarl³] cyclosporin A: Compound 3

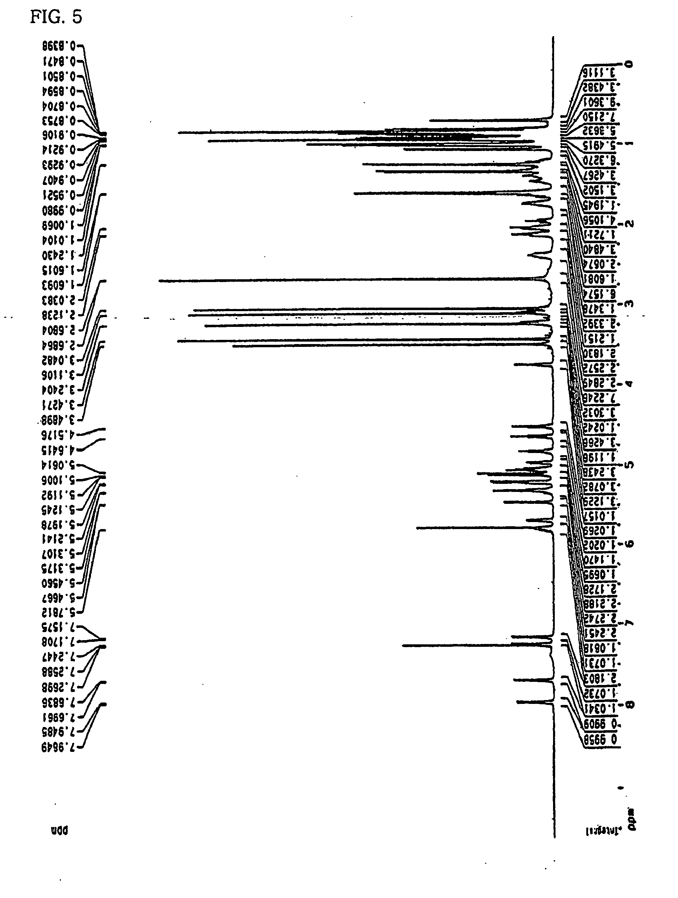

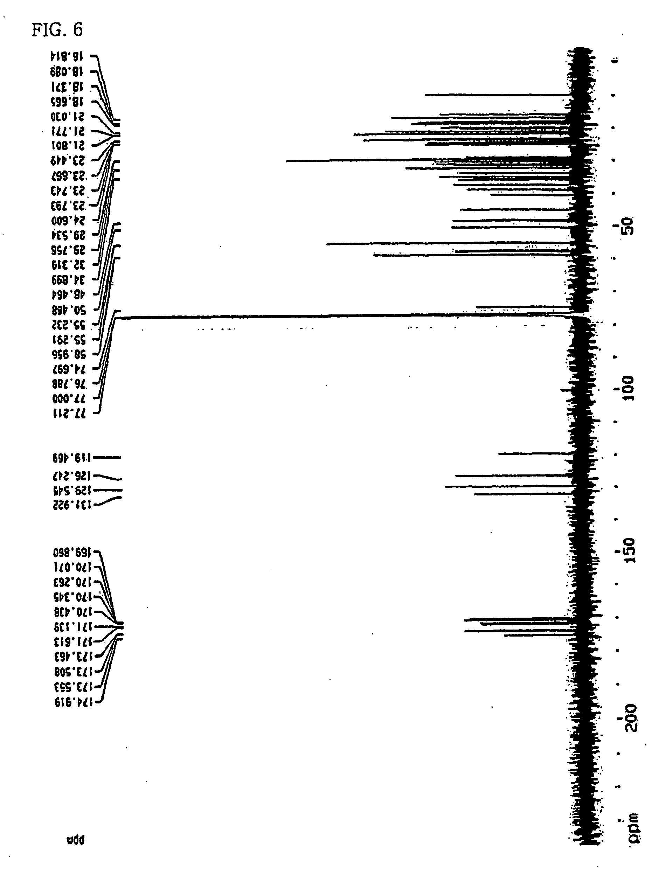

According to the general method above, THF (100 ml), (i-Pr)₂NH (1.6 ml), BuLi (4.0 ml), cyclosporin A (1.0 g) dissolved in THF (10 ml) and isopropyl disulfide (i-Pr₂S₂, 1.8 ml) were used. The reaction mixture was stirred for 24 hrs at 0 °C and added with 20 ml water, followed by concentration. The residue was added with ethyl acetate (EtOAc), washed with water and a solution of saturated sodium chloride in sequence, and dried over anhydrous MgSO₄. After concentrating, the residue was subjected to silica gel column chromatography (100 g silica gel, dichloromethane : methylalcohol = 100:1 ~ 50:1), followed by HPLC to give 0.12 g of the title compound. Molecular weight of the compound was determined by FAB MS (ZMS AX 505H) analysis. To confirm the molecular structure, Nuclear Magnetic Resonance (NMR) measurements were performed on 600 MHz (Bruker) for ¹H-NMR and on 150 MHz (Bruker) for ¹³C-NMR, and the spectra are shown in Figs. 5 and 6, respectively.

### 1-4: Synthesis of [D-2-allylthio-sar³] cyclosporin A: Compound 4

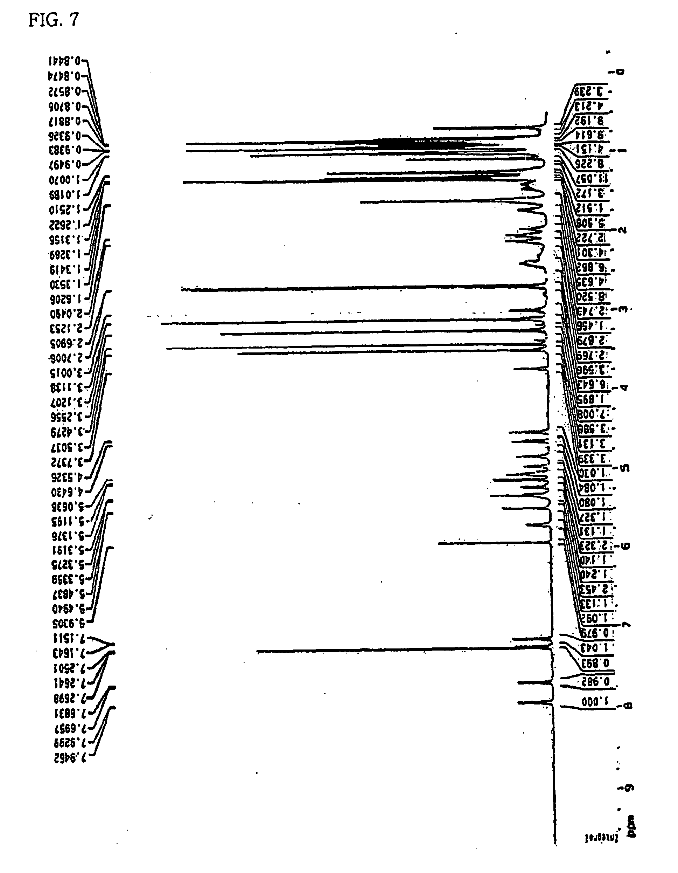

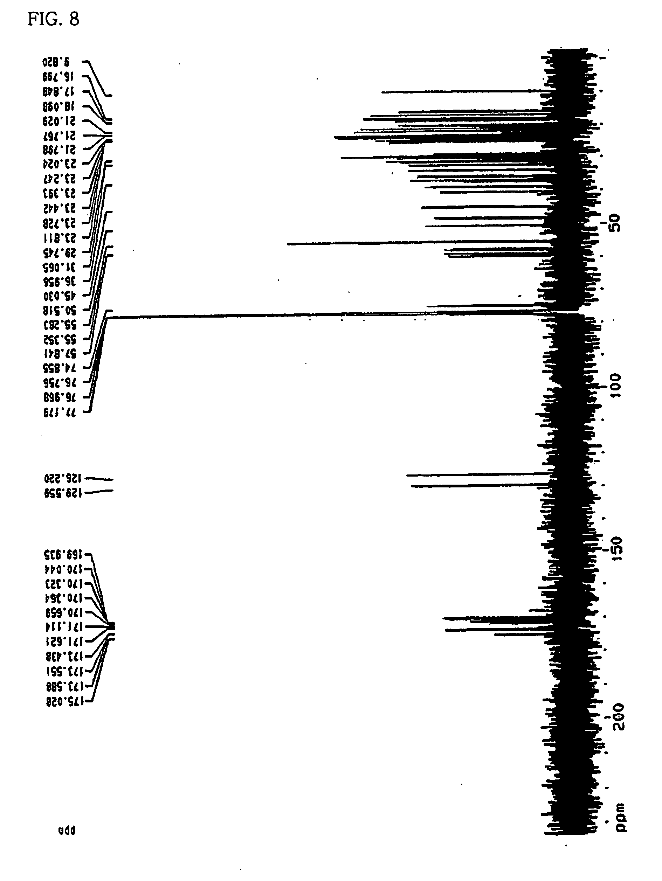

According to the general method above, THF (100 ml), (i-Pr)₂NH (1.6 ml), BuLi (4.0 ml), cyclosporin A (1.0 g) dissolved in THF (10 ml) and allyl disulfide ((CH₂=CHCH₂)₂S₂, 2.0 ml) were used. The reaction mixture was stirred for 24 hrs at 0 °C and added with 20 ml water, followed by concentration. The residue was added with ethyl acetate (EtOAc), washed with water and a solution , of saturated sodium chloride in sequence, and dried over anhydrous MgSO₄. After concentrating, the residue was subjected to silica gel column chromatography (100 g silica gel, dichloromethane : methylalcohol = 100:1 ~ 50: 1), followed by HPLC to give 0.15 g of the title compound. Molecular weight of the compound was determined by FAB MS (ZMS AX 505H) analysis. To confirm the molecular structure, Nuclear Magnetic Resonance (NMR) measurements were performed on 600 MHz (Bruker) for ¹H-NMR and on 150 MHz (Bruker) for ¹³C-NMR, and the spectra are shown in Figs. 7 and 8, respectively.

### 1-5: Synthesis of [D-2-benzylthio-sar³] cyclosporin A: Compound 5

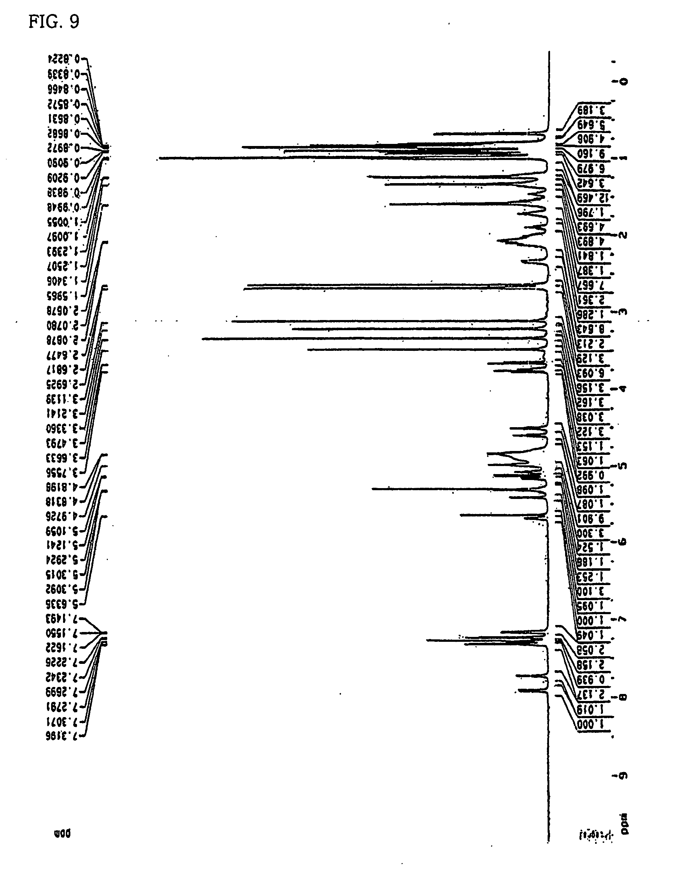

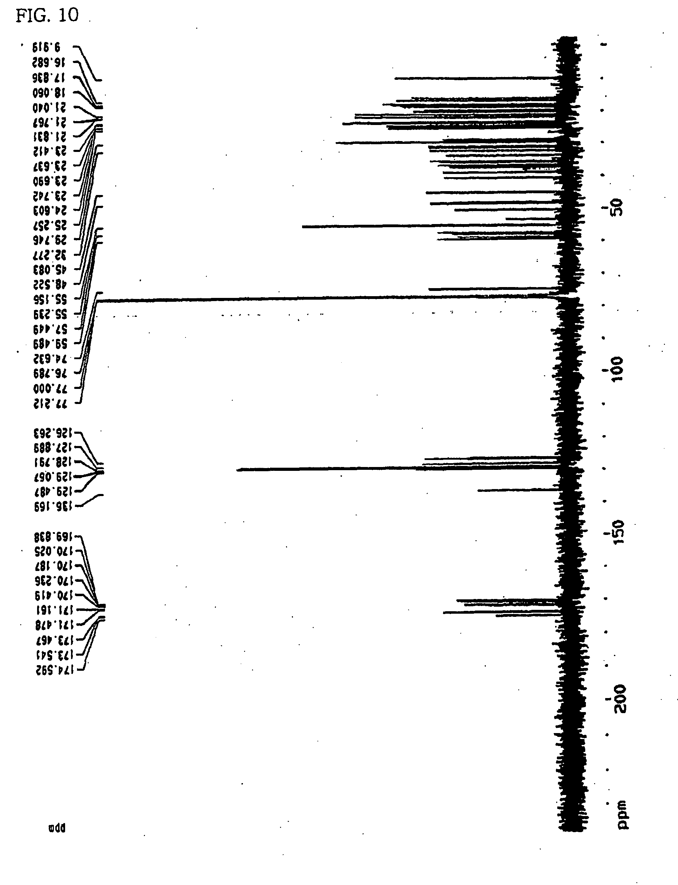

According to the general method above, THF (100 ml), (i-Pr)₂NH (1.6 ml), BuLi (4.0 ml), cyclosporin A (1.0 g) dissolved in THF (10 ml) and benzyl disulfide ((C₆H₅CH₂)₂S₂, 3 g) were used. The reaction mixture was stirred for 24 hrs at 0 °C and added with 20 ml water, followed by concentration. The residue was added with ethyl acetate (EtOAc), washed with water and a solution of saturated sodium chloride in sequence, and dried over anhydrous MgSO₄. After concentrating, the residue was subjected to silica gel column chromatography (100 g silica gel, dichloromethane : methylalcohol = 100:1 ~ 50:1), followed by HPLC to give 0.23 g of the title compound. Molecular weight of the compound was determined by FAB MS (ZMS AX 505H) analysis. To confirm the molecular structure, Nuclear Magnetic Resonance (NMR) measurements were performed on 600 MHz (Bruker) for ¹H-NMR and on 150 MHz (Bruker) for ¹³C-NMR, and the spectra are shown in Figs. 9 and 10, respectively.

### 1-6: Synthesis of [D-2-(4-nitrophenyl)thio-sar³] cyclosporin A: Compound 6

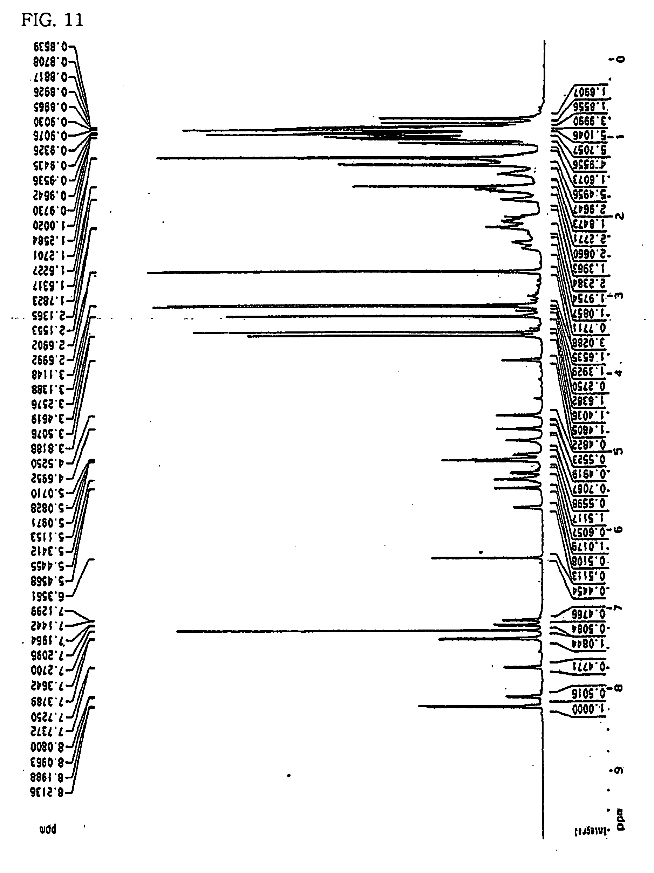

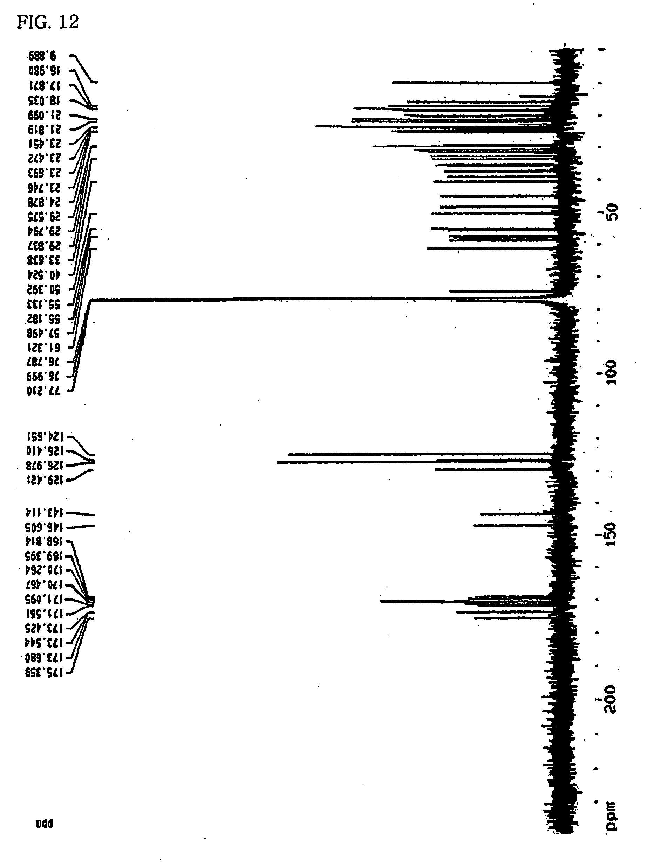

According to the general method above, THF (100 ml), (i-Pr)₂NH (1.6 ml), BuLi (4.0 ml), cyclosporin A (1.0 g) dissolved in THF (10 ml) and (4-nitrophenyl) disulfide ((4-NO₂C₆H₅)₂S₂, 4.2 g) were used. The reaction mixture was stirred for 24 hrs at 0 °C and added with 20 ml water, followed by concentration. The residue was added with ethyl acetate (EtOAc), washed with water and a solution of saturated sodium chloride in sequence, and dried over anhydrous MgSO₄. After concentrating, the residue was subjected to silica gel column chromatography (100 g silica gel, dichloromethane : methylalcohol = 100:1 ~ 50:1), followed by HPLC to give 0.11 g of the title compound. Molecular weight of the compound was determined by FAB MS (ZMS AX 505H) analysis. To confirm the molecular structure, Nuclear Magnetic Resonance (NMR) measurements were performed on 600 MHz (Bruker) for ¹H-NMR and on 150 MHz (Bruker) for ¹³C-NMR, and the spectra are shown in Figs. 11 and 12, respectively.

### 1-7: Synthesis of [D-2-(dimethylthiocarbamyl)dithio-sar³] cyclosporin A: Compound 7

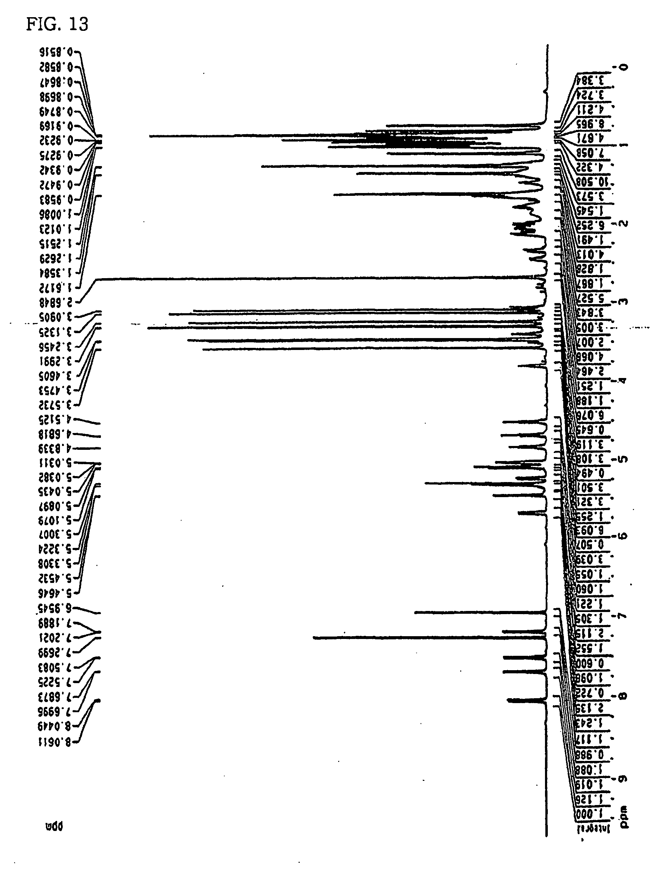

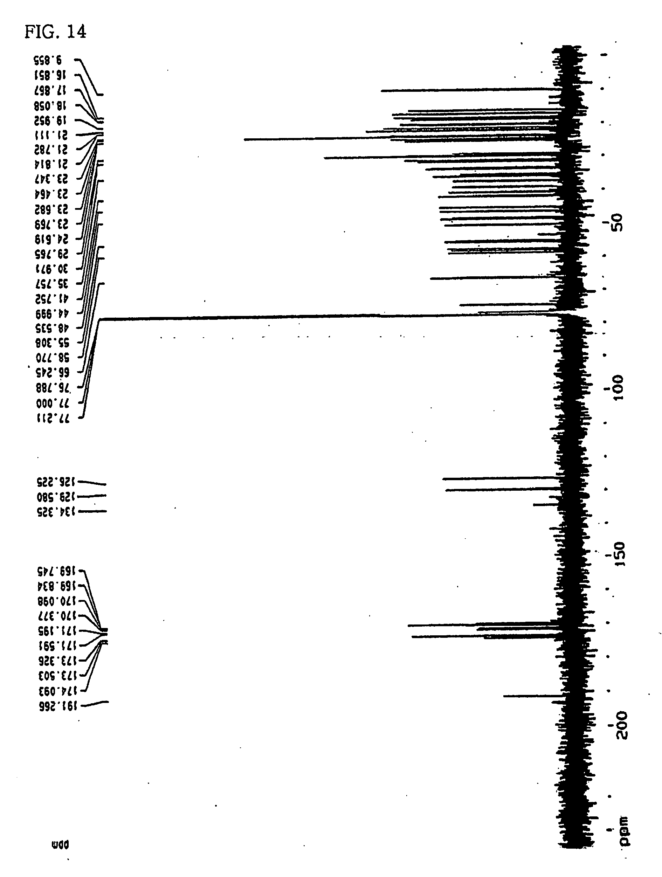

According to the general method above, THF (100 ml), (i-Pr)₂NH (1.6 ml), BuLi (4.0 ml), cyclosporin A (1.0 g) dissolved in THF (10 ml) and (tetramethylthiuram) disulfide (((CH₃)₂NCS₂)₂, 2.9 g) were used. The reaction mixture was stirred for 24 hrs at 0 °C and added with 20 ml water, followed by concentration. The residue was added with ethyl acetate (EtOAc), washed with water and a solution of saturated sodium chloride in sequence, and dried over anhydrous MgSO₄. After concentrating, the residue was subjected to silica gel column chromatography (100 g silica gel, dichloromethane : methylalcohol = 100:1 ~ 50:1), followed by HPLC to give 0.09 g of the title compound. Molecular weight of the compound was determined by FAB MS (ZMS AX 505H) analysis. To confirm the molecular structure, Nuclear Magnetic Resonance (NMR) measurements were performed on 600 MHz (Bruker) for ¹H-NMR and on 150 MHz (Bruker) for ¹³C-NMR, and the spectra are shown in Figs. 13 and 14, respectively.

### Preparative example 1: hair tonic

### 1-1: Preparation of hair tonic containing [D-2-ethylthio-sar³] cyclosporin A

Individual ingredients were mixed and stirred, and the mixtures were completely dissolved to prepare three hair growth promoting tonics, with compositions as shown in Table 1 below. It was found that the composition 1 of Table 1 has a hair growth promoting effect at a level similar to a conventional hair tonic containing 0.1 % cyclosporin A, as evaluated in an animal experiment according to the Test Example described later.

**Table 1: Formulation of hair tonic**

| (unit: weight %) | | | |
|---|---|---|---|
| Ingredients | Comp. 1 | Comp. 2 | Comp. 3 |
| Ethanol | 40.0 | 40.0 | 40.0 |
| [D-2-ethylthio-sar³] cyclosporin A | 0.1 | 1.0 | 8.0 |
| Tocopherol acetate | 0.1 | 0.1 | 0.1 |
| Salicylic acid | 0.3 | 0.3 | 0.3 |
| L-menthol | 0.3 | 0.3 | 0.3 |
| Tween 20 | 0.5 | 0.5 | 0.5 |
| Flavor | typical | typical | typical |
| Colarant | typical | typical | typical |
| Water | balance | balance | balance |

### 1-2: Preparation of hair tonic containing [D-2-propylthio-sar³] cyclosporin A

Individual ingredients were mixed and stirred, and the mixtures were completely dissolved to prepare three hair growth promoting tonics, with compositions as shown in Table 2 below. It was found that the composition 1 of Table 2 has a hair growth promoting effect at a level similar to a conventional hair tonic containing 0.1 % cyclosporin A, as evaluated in an animal experiment according to the Test Example described later.

**Table 2: Formulation of hair tonic**

| (unit: weight %) | | | |
|---|---|---|---|
| Ingredients | Comp. 1 | Comp. 2 | Comp. 3 |
| Ethanol | 40.0 | 40.0 | 40.0 |
| [D-2-propylthio-sar³] cyclosporin A | 0.1 | 1.0 | 8.0 |
| Tocopherol acetate | 0.1 | 0.1 | 0.1 |
| Salicylic acid | 0.3 | 0.3 | 0.3 |
| L-menthol | 0.3 | 0.3 | 0.3 |
| Tween 20 | 0.5 | 0.5 | 0.5 |
| Flavor | typical | typical | typical |
| Colarant | typical | typical | typical |
| Water | balance | balance | balance |

### 1-3: Preparation of hair tonic containing [D-2-isopropylthio-sar³] cyclosporin A

Individual ingredients were mixed and stirred, and the mixtures were completely dissolved to prepare three hair growth promoting tonics, with compositions as shown in Table 3 below. It was found that the composition 1 of Table 3 has a hair growth promoting effect at a level similar to a conventional hair tonic containing 0.1 % cyclosporin A, as evaluated in an animal experiment according to the Test Example described later.

**Table 3: Formulation of hair tonic**

| (unit: weight %) | | | |
|---|---|---|---|
| Ingredients | Comp. 1 | Comp.2 | Comp.3 |
| Ethanol | 40.0 | 40.0 | 40.0 |
| [D-2-isopropylthio-sa³] cyclosporin A | 0.1 | 1.0 | 8.0 |
| Tocopherol acetate | 0.1 | 0.1 | 0.1 |
| Salicylic acid | 0.3 | 0.3 | 0.3 |
| L-menthol | 0.3 | 0.3 | 0.3 |
| Tween 20 | 0.5 | 0.5 | 0.5 |
| Flavor | typical | typical | typical |
| Colarant | typical | typical | typical |
| Water | balance | balance | balance |

### 1-4: Preparation of hair tonic containing [D-2-allylthio-sar³] cyclosporin A

Individual ingredients were mixed and stirred, and the mixtures were completely dissolved to prepare three hair growth promoting tonics, with compositions as shown in Table 4 below. It was found that the composition 1 of Table 4 has a hair growth promoting effect at a level similar to a conventional hair tonic containing 0.1 % cyclosporin A, as evaluated in an animal experiment according to the Test Example described later.

**Table 4: Formulation of hair tonic**

| | | | |
|---|---|---|---|
| | | | |

| (unit: weight %) | | | |
|---|---|---|---|
| Ingredients | Comp. 1 | Comp. 2 | Comp. 3 |
| Ethanol | 40.0 | 40.0 | 40.0 |
| [D-2-allylthio-sar³] cyclosporin A | 0.1 | 1.0 | 8.0 |
| Tocopherol acetate | 0.1 | 0.1 | 0.1 |
| Salicylic acid | 0.3 | 0.3 | 0.3 |
| L-menthol | 0.3 | 0.3 | 0.3 |
| Tween 20 | 0.5 | 0.5 | 0.5 |
| Flavor | typical | typical | typical |
| Colarant | typical | typical | typical |
| Water | balance | balance | balance |

### 1-5: Preparation of hair tonic containing [D-2-benzylthio-sar³] cyclosporin A

Individual ingredients were mixed and stirred, and the mixtures were completely dissolved to prepare three hair growth promoting tonics, with compositions as shown in Table 5 below. It was found that the composition 1 of Table 5 has a hair growth promoting effect at a level similar to a conventional hair tonic containing 0.1 % cyclosporin A, as evaluated in an animal experiment according to the Test Example described later.

**Table 5: Formulation of hair tonic**

| (unit: weight %) | | | |
|---|---|---|---|
| Ingredients | Comp. 1 | Comp. 2 | Comp. 3 |
| Ethanol | 40.0 | 40.0 | 40.0 |
| [D-2-benzylthio-sar ³] cyclosporin A | 0.1 | 1.0 | 8.0 |
| Tocopherol acetate | 0.1 | 0.1 | 0.1 |
| Salicylic acid | 0.3 | 0.3 | 0.3 |
| L-menthol | 0.3 | 0.3 | 0.3 |
| Tween 20 | 0.5 | 0.5 | 0.5 |
| Flavor | typical | typical | typical |
| Colarant | typical | typical | typical |
| Water | balance | balance | balance |

### 1-6: Preparation of hair tonic containing [D-2-(4-nitrophenyl)thio-sar³] cyclosporins A

Individual ingredients were mixed and stirred, and the mixtures were completely dissolved to prepare three hair growth promoting tonics, with compositions as shown in Table 6 below. It was found that the composition 1 of Table 6 has a hair growth promoting effect at a level similar to a conventional hair tonic containing 0.1 % cyclosporin A, as evaluated in an animal experiment according to the Test Example described later.

**Table 6: Formulation of hair tonic**

| (unit: weight %) | | | |
|---|---|---|---|
| Ingredients | Comp. 1 | Comp. 2 | Comp. 3 |
| Ethanol | 40.0 | 40.0 | 40.0 |
| [D-2-(4-nitrophenyl)thio-sar³) cyclosporin A | 0.1 | 1.0 | 8.0 |
| Tocopherol acetate | 0.1 | 0.1 | 0.1 |
| Salicylic acid | 0.3 | 0.3 | 0.3 |
| L-menthol | 0.3 | 0.3 | 0.3 |
| Tween 20 | 0.5 | 0.5 | 0.5 |
| Flavor | typical | typical | typical |
| Colarant | typical | typical | typical |
| Water | balance | balance | balance |

### 1-7: Preparation of hair tonic containing [D-2-(dimethylthiocarbamyl) dithio-sar³] cyclosporin A

Individual ingredients were mixed and stirred, and the mixtures were completely dissolved to prepare three hair growth promoting tonics, with compositions as shown in Table 7 below. It was found that the composition 1 of Table 7 has a hair growth promoting effect at a level similar to a conventional hair tonic containing 0.1 % cyclosporin A, as evaluated in an animal experiment according to the Test Example described later.

**Table 7: Formulation of hair tonic**

| (unit: weight %) | | | |
|---|---|---|---|
| Ingredients | Comp. 1 | Comp. 2 | Comp. 3 |
| Ethanol | 40.0 | 40.0 | 40.0 |
| [D-2-(dimethylthiocarbamyl)dithio-sar³] cyclosporin A | 0.1 | 1.0 | 8.0 |
| Tocopherol acetate | 0.1 | 0.1 | 0.1 |
| Salicylic acid | 0.3 | 0.3 | 0.3 |
| L-menthol | 0.3 | 0.3 | 0.3 |
| Tween 20 | 0.5 | 0.5 | 0.5 |
| Flavor | typical | typical | typical |
| Colarant | typical | typical | typical |
| Water | balance | balance | balance |

### Preparative example 2: hair cream

### 2-1: Preparation of hair cream containing [D-2-ethylthio-sar³] cyclosporin A

Individual oil-phase and water-phase ingredients were mixed in a separate container, and each mixture was completely dissolved by heating to 80 °C. Two phases of the ingredients were mixed, emulsified, and cooled to room temperature. Additives such as flavor and colorant were admixed to prepare three hair creams, with compositions as shown in Table 8 below. Water was added to adjust to 100 % the total weight including the oil-phase and water-phase ingredients.

It was found that the composition 1 of Table 8 has a hair growth promoting effect at a level similar to a conventional hair cream containing 0.1 % cyclosporin A, as evaluated in an animal experiment according to the Test Example described later.

**Table 8: Formulation of hair cream**

| (unit: weight %) | | | |
|---|---|---|---|
| Ingredients | Comp.1 | Comp.2 | Comp.3 |
| Paraffin | 5.0 | 5.0 | 5.0 |
| Cetostearyl alcohol | 5.5 | 5.5 | 5.5 |
| Petrolatum | 5.5 | 5.5 | 5.5 |
| Glycerin monostearate | 3.0 | 3.0 | 3.0 |
| Polyoxyethyleneoctyldodecylether | 3.0 | 3.0 | 3.0 |
| Propylparaben | 0.3 | 0.3 | 0.3 |
| [D-2-ethylthio-sar³] cyclosporin A | 0.1 | 1.0 | 8.0 |
| Glycerin | 7.0 | 7.0 | 7.0 |
| Dipropyleneglycol | 20.0 | 20.0 | 20.0 |
| Polyethyleneglycol | 5.0 | 5.0 | 5.0 |
| Water | balance not including flavor and colorant | | |
| Flavor | typical | typical | typical |
| Colorant | typical | typical | typical |

### 2-2: Preparation of hair cream containing [D-2-propylthio-sar³] cyclosporin A

Individual oil-phase and water-phase ingredients were mixed in a separate container, and each mixture was completely dissolved by heating to 80 °C. Two phases of the ingredients were mixed, emulsified, and cooled to room temperature. Additives such as flavor and colorant were admixed to prepare three hair creams, with compositions as shown in Table 9 below. Water was added to adjust to 100 % the total weight including the oil-phase and water-phase ingredients.

It was found that the composition 1 of Table 9 has a hair growth promoting effect at a level similar to a conventional hair cream containing 0.1 % cyclosporin A, as evaluated in an animal experiment according to the Test Example described later.

**Table 9: Formulation of hair cream**

| (unit: weight %) | | | |
|---|---|---|---|
| Ingredients | Comp. 1 | Comp. 2 | Comp. 3 |
| Paraffin | 5.0 | 5.0 | 5.0 |
| Cetostearyl alcohol | 5.5 | 5.5 | 5.5 |
| Petrolatum | 5.5 | 5.5 | 5.5 |
| Glycerin monostearate | 3.0 | 3.0 | 3.0 |
| Polyoxyethyleneoctyldodecylether | 3.0 | 3.0 | 3.0 |
| Propylparaben | 0.3 | 0.3 | 0.3 |
| [D-2-propylthio-sar³] cyclosporin A | 0.1 | 1.0 | 8.0 |
| Glycerin | 7.0 | 7.0 | 7.0 |
| Dipropyleneglycol | 20.0 | 20.0 | 20.0 |
| Polyethyleneglycol | 5.0 | 5.0 | 5.0 |
| Water | balance not including flavor and colorant | | |
| Flavor | typical | typical | typical |
| Colorant | typical | typical | typical |

### 2-3: Preparation of hair cream containing [D-2-isopropylthio-sar³] cyclosporin A

Individual oil-phase and water-phase ingredients were mixed in a separate container, and each mixture was completely dissolved by heating to 80 °C. Two phases of the ingredients were mixed, emulsified, and cooled to room temperature. Additives such as flavor and colorant were admixed to prepare three hair creams, with compositions as shown in Table 10 below. Water was added to adjust to 100 % the total weight including the oil-phase and water-phase ingredients.

It was found that the composition 1 of Table 10 has a hair growth promoting effect at a level similar to a conventional hair cream containing 0.1 % cyclosporin A, as evaluated in an animal experiment according to the Test Example described later

**Table 10: Formulation of hair cream**

| (unit: weight %) | | | |
|---|---|---|---|
| Ingredients | Comp. 1 | Comp. 2 | Comp. 3 |
| Paraffin | 5.0 | 5.0 | 5.0 |
| Cetostearyl alcohol | 5.5 | 5.5 | 5.5 |
| Petrolatum | 5.5 | 5.5 | 5.5 |
| Glycerin monostearate | 3.0 | 3.0 | 3.0 |
| Polyoxyethyleneoctyldodecylether | 3.0 | 3.0 | 3.0 |
| Propylparaben | 0.3 | 0.3 | 0.3 |
| [D-2-isopropylthio-sar³]cyclosporin A | 0.1 | 1.0 | 8.0 |
| Glycerin | 7.0 | 7.0 | 7.0 |
| Dipropyleneglycol | 20.0 | 20.0 | 20.0 |
| Polyethyleneglycol | 5.0 | 5.0 | 5.0 |
| Water | balance not including flavor and colorant | | |
| Flavor | typical | typical | typical |
| Colorant | typical | typical | typical |

### 2-4: Preparation of hair cream containing [D-2-allylthio-sar³] cyclosporin A

Individual oil-phase and water-phase ingredients were mixed in a separate container, and each mixture was completely dissolved by heating to 80 °C. Two phases of the ingredients were mixed, emulsified, and cooled to room temperature. Additives such as flavor and colorant were admixed to prepare three hair creams, with compositions as shown in Table 11 below. Water was added to adjust to 100 % the total weight including the oil-phase and water-phase ingredients.

It was found that the composition 1 of Table 11 has a hair growth promoting effect at a level similar to a conventional hair cream containing 0.1 % cyclosporin A, as evaluated in an animal experiment according to the Test Example described later.

**Table 11: Formulation of hair cream**

| (unit: weight %) | | | |
|---|---|---|---|
| Ingredients | Comp. 1 | Comp. 2 | Comp. 3 |
| Paraffin | 5.0 | 5.0 | 5.0 |
| Cetostearyl alcohol | 5.5 | 5.5 | 5.5 |
| Petrolatum | 5.5 | 5.5 | 5.5 |
| Glycerin monostearate | 3.0 | 3.0 | 3.0 |
| Polyoxyethyleneoctyldodecylether | 3.0 | 3.0 | 3.0 |
| Propylparaben | 0.3 | 0.3 | 0.3 |
| [D-2-allytthio-sar³] cyclosporin A | 0.1 | 1.0 | 8.0 |
| Glycerin | 7.0 | 7.0 | 7.0 |
| Dipropyleneglycol | 20.0 | 20.0 | 20.0 |
| Polyethyleneglycol | 5.0 | 5.0 | 5.0 |
| Water | balance not including flavor and colorant | | |
| Flavor | typical | typical | typical |
| Colorant | typical | typical | typical |

### 2-5: Preparation of hair cream containing [D-2-benzylthio-sar³] cyclosporin A

Individual oil-phase and water-phase ingredients were mixed in a separate container, and each mixture was completely dissolved by heating to 80 °C. Two phases of the ingredients were mixed, emulsified, and cooled to room temperature. Additives such as flavor and colorant were admixed to prepare three hair creams, with compositions as shown in Table 12 below. Water was added to adjust to 100 % the total weight including the oil-phase and water-phase ingredients.

It was found that the composition 1 of Table 12 has a hair growth promoting effect at a level similar to a conventional hair cream containing 0.1 % cyclosporin A, as evaluated in an animal experiment according to the Test Example described later.

**Table 12: Formulation of hair cream**

| (unit: weight %) | | | |
|---|---|---|---|
| Ingredients | Comp. 1 | Comp. 2 | Comp. 3 |
| Paraffin | 5.0 | 5.0 | 5.0 |
| Cetostearyl alcohol | 5.5 | 5.5 | 5.5 |
| Petrolatum | 5.5 | 5.5 | 5.5 |
| Glycerin monostearate | 3.0 | 3.0 | 3.0 |
| Polyoxyethyleneoctyldodecylether | 3.0 | 3.0 | 3.0 |
| Propylparaben | 0.3 | 0.3 | 0.3 |
| [D-2-benzylthio-sar³] cyclosporin A | 0.1 | 1.0 | 8.0 |
| Glycerin | 7.0 | 7.0 | 7.0 |
| Dipropyleneglycol | 20.0 | 20.0 | 20.0 |
| Polyethyleneglycol | 5.0 | 5.0 | 5.0 |
| Water | balance not including flavor and colorant | | |
| Flavor | typical | typical | typical |
| Colorant | typical | typical | typical |

### 2-6: Preparation of hair cream containing [D-2-(4-nitrophenyl)thio-sar³] cyclosporin A

Individual oil-phase and water-phase ingredients were mixed in a separate container, and each mixture was completely dissolved by heating to 80 °C. Two phases of the ingredients were mixed, emulsified, and cooled to room temperature. Additives such as flavor and colorant were admixed to prepare three hair creams, with compositions as shown in Table 13 below. Water was added to adjust to 100 % the total weight including the oil-phase and water-phase ingredients.

It was found that the composition 1 of Table 13 has a hair growth promoting effect at a level similar to a conventional hair cream containing 0.1 % cyclosporin A, as evaluated in an animal experiment according to the Test Example described later.

**Table 13: Formulation of hair cream**

| (unit: weight %) | | | |
|---|---|---|---|
| Ingredients | Comp. 1 | Comp. 2 | Comp. 3 |
| Paraffin | 5.0 | 5.0 | 5.0 |
| Cetostearyl alcohol | 5.5 | 5.5 | 5.5 |
| Petrolatum | 5.5 | 5.5 | 5.5 |
| Glycerin monostearate | 3.0 | 3.0 | 3.0 |
| Polyoxyethyleneoctyldodecylether | 3.0 | 3.0 | 3.0 |
| Propylparaben | 0.3 | 0.3 | 0.3 |
| [D-2-(4-nitrophenyl)thio-sar³] cyclosporin A | 0.1 | 1.0 | 8.0 |
| Glycerin | 7.0 | 7.0 | 7.0 |
| Dipropyleneglycol | 20.0 | 20.0 | 20.0 |
| Polyethyleneglycol | 5.0 | 5.0 | 5.0 |
| Water | balance not including flavor and colorant | | |
| Flavor | typical | typical | typical |
| Colorant | typical | typical | typical |

### 2-7: Preparation of hair cream containing [D-2-(dimethylthiocarbamyl) dithio-sar³] cyclosporin A

Individual oil-phase and water-phase ingredients were mixed in a separate container, and each mixture was completely dissolved by heating to 80 °C. Two phases of the ingredients were mixed, emulsified, and cooled to room temperature. Additives such as flavor and colorant were admixed to prepare three hair cream, with compositions as shown in Table 14 below. Water was added to adjust to 100 % the total weight including the oil-phase and water-phase ingredients.

It was found that the composition 1 of Table 14 has a hair growth promoting effect at a level similar to a conventional hair cream containing 0.1 % cyclosporin A, as evaluated in an animal experiment according to the Test Example described later.

**Table 14: Formulation of hair cream**

| (unit: weight %) | | | |
|---|---|---|---|
| Ingredients | Comp. 1 | Comp. 2 | Comp. 3 |
| Paraffin | 5.0 | 5.0 | 5.0 |
| Cetostearyl alcohol | 5.5 | 5.5 | 5.5 |
| Petrolatum | 5.5 | 5.5 | 5.5 |
| Glycerin monostearate | 3.0 | 3.0 | 3.0 |
| Polyoxyethyleneoctyldodecylether | 3.0 | 3.0 | 3.0 |
| Propylparaben | 0.3 | 0.3 | 0.3 |
| [D-2-(dimethylthiocarbamyl)dithio-sar³]cyclosporin A | 0.1 | 1.0 | 8.0 |
| Glycerin | 7.0 | 7.0 | 7.0 |
| Dipropyleneglycol | 20.0 | 20.0 | 20.0 |
| Polyethyleneglycol | 5.0 | 5.0 | 5.0 |
| Water | balance not including flavor and colorant | | |
| Flavor | typical | typical | typical |
| Colorant | typical | typical | typical |

### Preparative example 3: shampoo

### 3-1: Preparation of shampoo containing [D-2-ethylthio-sar³] cyclosporin A

All individual ingredients, except flavor, colorant and water, were mixed and the mixture was completely dissolved by heating, while stirring. After cooling to room temperature, the mixture was mixed with flavor and colorant. Water was finally added to adjust to 100 % the total weight, to prepare three shampoos, with compositions as shown in Table 15 below.

**Table 15: Formulation of shampoo**

| (unit: weight %) | | | |
|---|---|---|---|
| Ingredients | Comp. 1 | Comp. 2 | Comp. 3 |
| Sodium POE lauryl sulfate (30%) | 40.0 | 40.0 | 40.0 |
| Palm oil fatty acid diethanolamide | 3.0 | 3.0 | 3.0 |
| Propyleneglycol | 2.0 | 2.0 | 2.0 |
| Methyl paraoxybenzoic acid | 0.2 | 0.2 | 0.2 |
| Ethanol | 2.0 | 2.0 | 2.0 |
| [D-2-ethylthio-sar³] cyclosporin A | 1.0 | 3.0 | 10.0 |
| Salicylic acid | 0.3 | 0.3 | 0.3 |
| L-menthol | 0.3 | 0.3 | 0.3 |
| Flavor | typical | typical | typical |
| Colorant | typical | typical | typical |
| Water | balance | balance | balance |

### 3-2: Preparation of shampoo containing [D-2-propylthio-sar³] cyclosporin A

All individual ingredients, except flavor, colorant and water, were mixed and the mixture was completely dissolved by heating, while stirring. After cooling to room temperature, the mixture was mixed with flavor and colorant. Water was finally added to adjust to 100 % the total weight, to prepare three shampoos, with compositions as shown in Table 16 below.

**Table 16: Formulation of shampoo**

| (unit: weight %) | | | |
|---|---|---|---|
| Ingredients | Comp. 1 | Comp. 2 | Comp. 3 |
| Sodium POE lauryl sulfate (30%) | 40.0 | 40.0 | 40.0 |
| Palm oil fatty acid diethanolamide | 3.0 | 3.0 | 3.0 |
| Propyleneglycol | 2.0 | 2.0 | 2.0 |
| Methyl paraoxybenzoic acid | 0.2 | 0.2 | 0.2 |
| Ethanol | 2.0 | 2.0 | 2.0 |
| [D-2-propylthio-sar³] cyclosporin A | 1.0 | 3.0 | 10.0 |
| Salicylic acid | 0.3 | 0.3 | 0.3 |
| L-menthol | 0.3 | 0.3 | 0.3 |
| Flavor | typical | typical | typical |
| Colorant | typical | typical | typical |
| Water | balance | balance | balance |

### 3-3: Preparation of shampoo containing [D-2-isopropylthio-sar³] cyclosporin A

All individual ingredients, except flavor, colorant and water, were mixed and the mixture was completely dissolved by heating, while stirring. After cooling to room temperature, the mixture was mixed with flavor and colorant. Water was finally added to adjust to 100 % the total weight, to prepare three shampoos, with compositions as shown in Table 17 below.

**Table 17: Formulation of shampoo**

| (unit: weight %) | | | |
|---|---|---|---|
| Ingredients | Comp. 1 | Comp. 2 | Comp. 3 |
| Sodium POE lauryl sulfate (30%) | 40.0 | 40.0 | 40.0 |
| Palm oil fatty acid diethanolamide | 3.0 | 3.0 | 3.0 |
| Propyleneglycol | 2.0 | 2.0 | 2.0 |
| Methyl paraoxybenzoic acid | 0.2 | 0.2 | 0.2 |
| Ethanol | 2.0 | 2.0 | 2.0 |
| [D-2-isopropylthio-sa³] cyclosporin A | 1.0 | 3.0 | 10.0 |
| Salicylic acid | 0.3 | 0.3 | 0.3 |
| L-menthol | 0.3 | 0.3 | 0.3 |
| Flavor | typical | typical | typical |
| Colorant | typical | typical | typical |
| Water | balance | balance | balance |

### 3-4: Preparation of shampoo containing [D-2-allylthio-sar³] cyclosporin A

All individual ingredients, except flavor, colorant and water, were mixed and the mixture was completely dissolved by heating, while stirring. After cooling to room temperature, the mixture was mixed with flavor and colorant. Water was finally added to adjust to 100 % the total weight, to prepare three shampoos, with compositions as shown in Table 18 below.

**Table 18: Formulation of shampoo**

| (unit: weight %) | | | |
|---|---|---|---|
| Ingredients | Comp. 1 | Comp. 2 | Comp. 3 |
| Sodium POE lauryl sulfate (30%) | 40.0 | 40.0 | 40.0 |
| Palm oil fatty acid diethanolamide | 3.0 | 3.0 | 3.0 |
| Propyleneglycol | 2.0 | 2.0 | 2.0 |
| Methyl paraoxybenzoic acid | 0.2 | 0.2 | 0.2 |
| Ethanol | 2.0 | 2.0 | 2.0 |
| [D-2-allylthio-sar³] cyclosporin A | 1.0 | 3.0 | 10.0 |
| Salicylic acid | 0.3 | 0.3 | 0.3 |
| L-menthol | 0.3 | 0.3 | 0.3 |
| Flavor | typical | typical | typical |
| Colorant | typical | typical | typical |
| Water | balance | balance | balance |

### 3-5: Preparation of shampoo containing [D-2-benzylthio-sar³] cyclosporin A

All individual ingredients, except flavor, colorant and water, were mixed and the mixture was completely dissolved by heating, while stirring. After cooling to room temperature, the mixture was mixed with flavor and colorant. Water was finally added to adjust to 100 % the total weight, to prepare three shampoos, with compositions as shown in Table 19 below.

**Table 19: Formulation of shampoo**

| (unit: weight %) | | | |
|---|---|---|---|
| Ingredients | Comp. 1 | Comp. 2 | Comp. 3 |
| Sodium POE lauryl sulfate (30%) | 40.0 | 40.0 | 40.0 |
| Palm oil fatty acid diethanolamide | 3.0 | 3.0 | 3.0 |
| Propyleneglycol | 2.0 | 2.0 | 2.0 |
| Methyl paraoxybenzoic acid | 0.2 | 0.2 | 0.2 |
| Ethanol | 2.0 | 2.0 | 2.0 |
| [D-2-benzylthio-sar³] cyclosporin A | 1.0 | 3.0 | 10.0 |
| Salicylic acid | 0.3 | 0.3 | 0.3 |
| L-menthol | 0.3 | 0.3 | 0.3 |
| Flavor | typical | typical | typical |
| Colorant | typical | typical | typical |
| Water | balance | balance | balance |

### 3-6: Preparation of shampoo containing [D-2-(4-nitrophenyl)thio-sar³] cyclosporin A

All individual ingredients, except flavor, colorant and water, were mixed and the mixture was completely dissolved by heating, while stirring. After cooling to room temperature, the mixture was mixed with flavor and colorant. Water was finally added to adjust to 100 % the total weight, to prepare three shampoos, with compositions as shown in Table 20 below.

**Table 20: Formulation of shampoo**

| (unit: weight %) | | | |
|---|---|---|---|
| Ingredients | Comp. 1 | Comp. 2 | Comp. 3 |
| Sodium POE lauryl sulfate (30%) | 40.0 | 40.0 | 40.0 |
| Palm oil fatty acid diethanolamide' | 3.0 | 3.0 | 3.0 |
| Propyleneglycol | 2.0 | 2.0 | 2.0 |
| Methyl paraoxybenzoic acid | 0.2 | 0.2 | 0.2 |
| Ethanol | 2.0 | 2.0 | 2.0 |
| [D-2-(4-nitrophenyl)thio-sar³] cyclosporin A | 1.0 | 3.0 | 10.0 |
| Salicylic acid | 0.3 | 0.3 | 0.3 |
| L-menthol | 0.3 | 0.3 | 0.3 |
| Flavor | typical | typical | typical |
| Colorant | typical | typical | typical |
| Water | balance | balance | balance |

### 3-7: Preparation of shampoo containing [D-2-(dimethylthiocarbamyl) dithio-sar³] cyclosporin A

All individual ingredients, except flavor, colorant and water, were mixed and the mixture was completely dissolved by heating, while stirring. After cooling to room temperature, the mixture was mixed with flavor and colorant. Water was finally added to adjust to 100 % the total weight, to prepare three shampoos, with compositions as shown in Table 21 below.

**Table 21: Formulation of shampoo**

| (unit: weight %) | | | |
|---|---|---|---|
| Ingredients | Comp. 1 | Comp. 2 | Comp. 3 |
| Sodium POE lauryl sulfate (30%) | 40.0 | 40.0 | 40.0 |
| Palm oil fatty acid diethanolamide | 3.0 | 3.0 | 3.0 |
| Propyleneglycol | 2.0 | 2.0 | 2.0 |
| Methyl paraoxybenzoic' acid | 0.2 | 0.2 | 0.2 |
| Ethanol | 2.0 | 2.0 | 2.0 |
| [D-2-(dimethylthiocarbamyl)dithio-sar³] cyclosporin A | 1.0 | 3.0 | 10.0 |
| Salicylic acid | 0.3 | 0.3 | 0.3 |
| L-menthol | 0.3 | 0.3 | 0.3 |
| Flavor | typical | typical | typical |
| Colorant | typical | typical | typical |
| Water | balance | balance | balance |

### Preparative example 4: hair conditioner

### 4-1: Preparation of hair conditioner containing [D-2-ethylthio-sar³] cyclosporin A

Individual oil-phase and water-phase ingredients were mixed in a separate container, and each mixture was completely dissolved by heating to 80 °C. Two phases of the ingredients were mixed, emulsified, and cooled to room temperature. Additives such as flavor and colorant were admixed to prepare three hair conditioners, with compositions as shown in Table 22 below. Water was added to adjust to 100 % the total weight including the oil-phase and water-phase ingredients.

**Table 22: Formulation of hair conditioner**

| (unit: weight %) | | | |
|---|---|---|---|
| Ingredients | Comp. 1 | Comp. 2 | Comp. 3 |
| Cetanol | 3.0 | 3.0 | 3.0 |
| Self-emulsifiable glycerol-monostearate | 2.0 | 2.0 | 3.0 |
| Squalene | 10.0 | 10.0 | 10.0 |
| [D-2-ethylthio-sar³] cyclosporin A | 1.0 | 5.0 | 10.0 |
| Propyleneglycol | 2.0 | 2.0 | 2.0 |
| Stearyldimethylbenzylammonium chloride (25%) | 8.0 | 8.0 | 8.0 |
| Methyl paraoxybenzoic acid | 0.2 | 0.2 | 0.2 |
| Salicylic acid | 0.3 | 0.3 | 0.3 |
| L-menthol | 0.3 | 0.3 | 0.3 |
| Water | balance | balance | balance |
| Flavor | typical | typical | typical |
| Colorant | typical | typical | typical |

### 4-2: Preparation of hair conditioner containing [D-2-propylthio-sar³] cyclosporin A

Individual oil-phase and water-phase ingredients were mixed in a separate container, and each mixture was completely dissolved by heating to 80 °C. Two phases of the ingredients were mixed, emulsified, and cooled to room temperature. Additives such as flavor and colorant were admixed to prepare three hair conditioners, with compositions as shown in Table 23 below. Water was added to adjust to 100 % the total weight including the oil-phase and water-phase ingredients.

**Table 23: Formulation of hair conditioner**

| (unit: weight %) | | | |
|---|---|---|---|
| Ingredients | Comp. 1 | Comp. 2 | Comp. 3 |
| Cetanol | 3.0 | 3.0 | 3.0 |
| Self-emulsifiable glycerol-monostearate | 2.0 | 2.0 | 3.0 |
| Squalene | 10.0 | 10.0 | 10.0 |
| [D-2-propylthio-sar³] cyclosporin A | 1.0 | 5.0 | 10.0 |
| Propyleneglycol | 2.0 | 2.0 | 2.0 |
| Stearyldimethylbenzylammonium chloride (25%) | 8.0 | 8.0 | 8.0 |
| Methyl paraoxybenzoic acid | 0.2 | 0.2 | 0.2 |
| Salicylic acid | 0.3 | 0.3 | 0.3 |
| L-menthol | 0.3 | 0.3 | 0.3 |
| Water | balance | balance | balance |
| Flavor | typical | typical | typical |
| Colorant | typical | typical | typical |

### 4-3: Preparation of hair conditioner containing [D-2-isopropylthio-sar³] cyclosporin A

Individual oil-phase and water-phase ingredients were mixed in a separate container, and each mixture was completely dissolved by heating to 80 °C. Two phases of the ingredients were mixed, emulsified, and cooled to room temperature. Additives such as flavor and colorant were admixed to prepare three hair conditioners, with compositions as shown in Table 24 below. Water was added to adjust to 100 % the total weight including the oil-phase and water-phase ingredients.

**Table 24: Formulation of hair conditioner**

| (unit: weight %) | | | |
|---|---|---|---|
| Ingredients | Comp. 1 | Comp. 2 | Comp. 3 |
| Cetanol | 3.0 | 3.0 | 3.0 |
| Self-emulsifiable glycerol-monostearate | 2.0 | 2.0 | 3.0 |
| Squalene | 10.0 | 10.0 | 10.0 |
| [D-2-isopropylthio-sar³] cyclosporin A | 1.0 | 5.0 | 10.0 |
| Propyleneglycol | 2.0 | 2.0 | 2.0 |
| Stearyldimethylbenzylammonium chloride (25%) | 8.0 | 8.0 | 8.0 |
| Methyl paraoxybenzoic acid | 0.2 | 0.2 | 0.2 |
| Salicylic acid | 0.3 | 0.3 | 0.3 |
| L-menthol | 0.3 | 0.3 | 0.3 |
| Water | balance | balance | balance |
| Flavor | typical | typical | typical |
| Colorant | typical | typical | typical |

### 4-4: Preparation of hair conditioner containing [D-2-allylthio-sar³] cyclosporin

Individual oil-phase and water-phase ingredients were mixed in a separate container, and each mixture was completely dissolved by heating to 80 °C. Two phases of the ingredients were mixed, emulsified, and cooled to room temperature. Additives such as flavor and colorant were admixed to prepare three hair conditioners, with compositions as shown in Table 25 below. Water was added to adjust to 100 % the total weight including the oil-phase and water-phase ingredients.

**Table 25: Formulation of hair conditioner**

| (unit: weight %) | | | |
|---|---|---|---|
| Ingredients | Comp. 1 | Comp. 2 | Comp. 3 |
| Cetanol | 3.0 | 3.0 | 3.0 |
| Self-emulsifiable glycerol-monostearate | 2.0 | 2.0 | 3.0 |
| Squalene | 10.0 | 10.0 | 10.0 |
| [D-2-allylthio-sar³]-cyclosporin A | 1.0 | 5.0 | 10.0 |
| Propyleneglycol | 2.0 | 2.0 | 2.0 |
| Stearyldimethylbenzylammonium chloride (25%) | 8.0 | 8.0 | 8.0 |
| Methyl paraoxybenzoic acid | 0.2 | 0.2 | 0.2 |
| Salicylic acid | 0.3 | 0.3 | 0.3 |
| L-menthol | 0.3 | 0.3 | 0.3 |
| Water | balance | balance | balance |
| Flavor | typical | typical | typical |
| Colorant | typical | typical | typical |

### 4-5: Preparation of hair conditioner containing [D-2-benzylthio-sar³] cyclosporin A

Individual oil-phase and water-phase ingredients were mixed in a separate container, and each mixture was completely dissolved by heating to 80 °C. Two phases of the ingredients were mixed, emulsified, and cooled to room temperature. Additives such as flavor and colorant were admixed to prepare three hair conditioners, with compositions as shown in Table 26 below. Water was added to adjust to 100 % the total weight including the oil-phase and water-phase ingredients.

**Table 26: Formulation of hair conditioner**

| (unit: weight %) | | | |
|---|---|---|---|
| Ingredients | Comp. 1 | Comp. 2 | Comp. 3 |
| Cetanol | 3.0 | 3.0 | 3.0 |
| Self-emulsifiable glycerol-monostearate | 2.0 | 2.0 | 3.0 |
| Squalene | 10.0 | 10.0 | 10.0 |
| [D-2-benzylthio-sar³] cyclosporin A | 1.0 | 5.0 | 10.0 |
| Propyleneglycol | 2.0 | 2.0 | 2.0 |
| Stearyldimethylbenzylammonium chloride (25%) | 8.0 | 8.0 | 8.0 |
| Methyl paraoxybenzoic acid | 0.2 | 0.2 | 0.2 |
| Salicylic acid | 0.3 | 0.3 | 0.3 |
| L-menthol | 0.3 | 0.3 | 0.3 |
| Water | balance | balance | balance |
| Flavor | typical | typical | typical |
| Colorant | typical | typical | typical |

### 4-6: Preparation of hair conditioner containing [D-2-(4-nitrophenyl)thiosar³] cyclosporin A

Individual oil-phase and water-phase ingredients were mixed in a separate container, and each mixture was completely dissolved by heating to 80 °C. Two phases of the ingredients were mixed, emulsified, and cooled to room temperature. Additives such as flavor and colorant were admixed to prepare three hair conditioners, with compositions as shown in Table 27 below. Water was added to adjust to 100 % the total weight including the oil-phase and water-phase ingredients.

**Table 27: Formulation of hair conditioner**

| (unit: weight %) | | | |
|---|---|---|---|
| Ingredients | Comp.1 | Comp. 2 | Comp. 3 |
| Cetanol | 3.0 | 3.0 | 3.0 |
| Self-emulsifiable glycerol-monostearate | 2.0 | 2.0 | 3.0 |
| Squalene | 10.0 | 10.0 | 10.0 |
| [D-2-(4-nitrophenyl)thio-sar³] cyclosporin A | 1.0 | 5.0 | 10.0 |
| Propyleneglycol | 2.0 | 2.0 | 2.0 |
| Stearyldimethylbenzylammonium chloride (25%) | 8.0 | 8.0 | 8.0 |
| Methyl paraoxybenzoic acid | 0.2 | 0.2 | 0.2 |
| Salicylic acid | 0.3 | 0.3 | 0.3 |
| L-menthol | 0.3 | 0.3 | 0.3 |
| Water | balance | balance | balance |
| Flavor | typical | typical | typical |
| Colorant | typical | typical | typical |

### 4-7: Preparation of hair conditioner containing [D-2-(dimethylthio carbamyl)dithio-sar³] cyclosporin A

Individual oil-phase and water-phase ingredients were mixed in a separate container, and each mixture was completely dissolved by heating to 80 °C. Two phases of the ingredients were mixed, emulsified, and cooled to room temperature. Additives such as flavor and colorant were admixed to prepare three hair conditioners, with compositions as shown in Table 28 below. Water was added to adjust to 100 % the total weight including the oil-phase and water-phase ingredients.

**Table 28: Formulation of hair conditioner**

| (unit: weight %) | | | |
|---|---|---|---|
| Ingredients | Comp. 1 | Comp. 2 | Comp. 3 |
| Cetanol | 3.0 | 3.0 | 3.0 |
| Self-emulsifiable glycerol-monostearate | 2.0 | 2.0 | 3.0 |
| Squalene | 10.0 | 10.0 | 10.0 |
| [D-2-(dimethylthiocarbamyl)dithio-sar³] cyclosporin A | 1.0 | 5.0 | 10.0 |
| Propyleneglycol | 2.0 | 2.0 | 2.0 |
| Stearyldimethylbenzylammonium chloride (25%) | 8.0 | 8.0 | 8.0 |
| Methyl paraoxybenzoic acid | 0.2 | 0.2 | 0.2 |
| Salicylic acid | 0.3 | 0.3 | 0.3 |
| L-menthol | 0.3 | 0.3 | 0.3 |
| Water | balance | balance | balance |
| Flavor | typical | typical | typical |
| Colorant | typical | typical | typical |

### Test Example: Test for hair growth promoting effect of cyclosporin derivatives of the invention

Female C57BL/6 mice of ages 6 to 7 weeks were utilized. After removing hairs on the middle of the back with an electric shaver, the mice were weighed and randomly assigned to the test groups with an even distribution of weights. The mice were given one day for adaptation. From the next day, mice were applied once a day on their backs with cyclosporin A and the cyclosporin A derivatives (Compounds 1 to 7) prepared by HPLC in Example 1 in amounts of 100 µl (conc. 0.1% w/v) for 30 days. The results were determined by visual approach, in terms of degrees of hair regrowth. With respect to respective hair-removed areas, rates of new hair growth were examined and compared.

As can be seen in Table 29, cyclosporin derivatives of the invention have a significant hair growth promoting effect, compared to the control in which mice were applied with a vehicle only. Further, the derivatives show a similar level of hair growth promoting effect, with respect to cyclosporin A. Meanwhile, over a course of 30 days, as comparing the appearance of the backs, the mice of the control and all test groups showed no specific skin irritation.

**Table 29: Evaluation of cyclosporin derivatives based on hair regrowth in mice**

| Compound applied | Vehicle | cyclosporin A | 1 | 2 | 3 | 4 | 5 | 6 | 7 |
|---|---|---|---|---|---|---|---|---|---|
| Area rate of hair regrowth (%) | 40 | 94 | 90 | 92 | 85 | 90 | 90 | 89 | 85 |

On the basis of the foregoing results, the cyclosporin derivatives of the invention may be formulated in any form including liquid formulations, sprays, gels, pastes, emulsions, creams, conditioners, shampoos, and the like. A variety of forms are available though, considering their high commercial demand, hair tonics, creams, conditioners, and shampoos are provided herein. As revealed in the above the Test Example, the cyclosporin derivatives exhibit an excellent hair growth promoting effect, compared to the control.

### Industrial Applicability

As apparent from the above description, the present invention provides a hair growth promoting agent comprising a cyclosporin A derivative substituted in the 3-position of cyclosporin A as an active ingredient, which exhibits an excellent hair growth promoting effect.

## Claims

1. A hair growth promoting agent comprising a 3-position analog of cyclosporin represented by Formula 1, as an active ingredient: 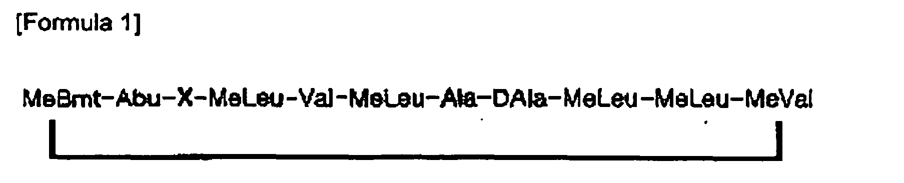 wherein:
MeBmt represents N-methyl-(4R)-4-[(E)-2-butenyl]-4-methyl-L-threonine;
Abu represents L-aminobutyric acid;
MeLeu represents N-methyl-L-leucine;
Val represents L-valine;
Ala represents L-alanine;
DAla represents D-alanine;
MeVal represents N-methyl-L-valine;
wherein X is [D-2-ethylthio-sarcosine], [D-2-propylthio-sarcosine], [D-2-isopropylthio-sarcosine], [D-2-allylthio-sarcosine], [D-2-benzylthio-sarcosine], [D-2-(4-nitrophenyl)thio-sarcosine] or [D-2-(dimethylthiocarbamyl)dithio-sarcosine].

2. The hair growth promoting agent as set forth in claim 1, comprising [D-2-ethylthio-sar3] cyclosporin A as an active ingredient.

3. The hair growth promoting agent as set forth in claim 1, comprising [D-2-propylthio-sar3] cyclosporin A as an active ingredient.

4. The hair growth promoting agent as set forth in claim 1, comprising [D-2-iopropylthio-sar3] cyclosporin A as an active ingredient.

5. The hair growth promoting agent as set forth in claim 1, comprising [D-2-allylthio-sar3] cyclosporin A as an active ingredient.

6. The hair growth promoting agent as set forth in claim 1, comprising [D-2-benzylthio-sar3] cyclosporin A as an active ingredient.

7. The hair growth promoting agent as set forth in claim 1, comprising [D-2-(4-nitrophenyl)thio-sar3] cyclosporin A as an active ingredient.

8. The hair growth promoting agent as set forth in claim 1, comprising [D-2-(dimethylthlocarbamyl)dithio-sar3] cyclosporin A as an active ingredient.

9. The hair growth promoting agent as set forth in any one of claims 1 to 8, which is formulated in a form selected from the group consisting of liquid formulation, spray, gel, paste, emulsion, cream, conditioner and shampoo.

## Patentansprüche

1. Den Haarwuchs förderndes Mittel, umfassend ein 3-Positions-Analogon von Cyclosporin, das durch die Formel 1 dargestellt wird, als einen aktiven Bestandteil: 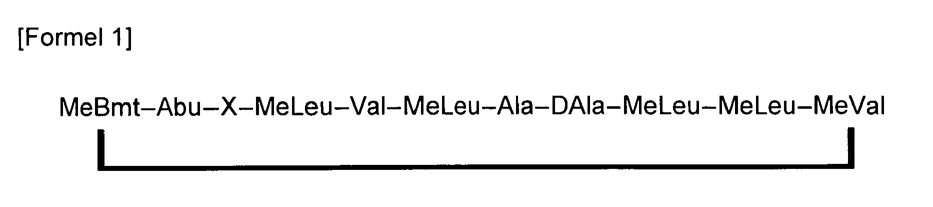 wobei:
MeBmt N-methyl-(4R)-4-[(E)-2-butenyl]-4-methyl-L-threonin darstellt;
Abu L-Aminobuttersäure darstellt;
MeLeu N-Methyl-L-leucin darstellt;
Val L-Valin darstellt;
Ala L-Alanin darstellt;
DAla D-Alanin darstellt;
MeVal N-Methyl-L-valin darstellt;
wobei X [D-2-Ethylthio-sarcosin], [D-2-Propylthio-sarcosin], [D-2-Isopropylthio-sarcosin], [D-2-Allylthio-sarcosin], [D-2-Benzylthio-sarcosin], [D-2-(4-Nitrophenyl)thio-sarcosin] oder [D-2-(Dimethylthiocarbamyl)dithio-sarcosin] ist.

2. Den Haarwuchs förderndes Mittel nach Anspruch 1, umfassend [D-2-Ethylthio-sar3]-cyclosporin A als einen aktiven Bestandteil.

3. Den Haarwuchs förderndes Mittel nach Anspruch 1, umfassend [D-2-Propylthio-sar3]-cyclosporin A als einen aktiven Bestandteil.

4. Den Haarwuchs förderndes Mittel nach Anspruch 1, umfassend [D-2-Isopropylthio-sar3]-cyclosporin A als einen aktiven Bestandteil.

5. Den Haarwuchs förderndes Mittel nach Anspruch 1, umfassend [D-2-Allylthio-sar3]-cyclosporin A als einen aktiven Bestandteil.

6. Den Haarwuchs förderndes Mittel nach Anspruch 1, umfassend [D-2-Benzylthio-sar3]-cyclosporin A als einen aktiven Bestandteil.

7. Den Haarwuchs förderndes Mittel nach Anspruch 1, umfassend [D-2-(4-Nitrophenyl)thio-sar3]-cyclosporin A als einen aktiven Bestandteil.

8. Den Haarwuchs förderndes Mittel nach Anspruch 1, umfassend [D-2-(Dimethylthiocarbamyl)-dithio-sar3]-cyclosporin A als einen aktiven Bestandteil.

9. Den Haarwuchs förderndes Mittel nach einem der Ansprüche 1 bis 8, welches in einer Form ausgewählt aus der Gruppe bestehend aus flüssige Formulierung, Spray, Gel, Paste, Emulsion, Creme, Conditioner oder Shampoo formuliert ist.

## Revendications

1. Agent de favorisation de la croissance capillaire comprenant un analogue de la cyclosporine substitue en position 3 représenté par la formule 1, comme principe actif : 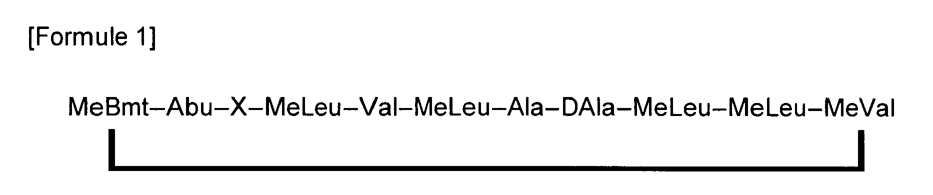 dans laquelle :
MeBmt représente la N-méthyl-(4R)-4-[(E)-2-butényl]-4-méthyl-L-thréonine ;
Abu représente l'acide L-aminobutyrique ;
MeLeu représente la N-méthyl-L-leucine ;
Val représente la L-valine ;
Ala représente la L-alanine ;
DAla représente la D-alanine ;
MeVal représente la N-méthyl-L-valine ;
dans lequel X est [D-2-éthylthio-sarcosine], [D-2-propylthio-sarcosine], [D-2-isopropylthio-sarcosine], [D-2-allylthio-sarcosine], [D-2-benzylthio-sarcosine], [D-2-(4-nitrophényl)thio-sarcosine] ou [D-2-(diméthylthiocarbamyl)dithio-sarcosine].

2. Agent de favorisation de la croissance capillaire tel que défini dans la revendication 1, comprenant la cyclosporine A [D-2-éthylthio-sar3] comme principe actif.

3. Agent de favorisation de la croissance capillaire tel que défini dans la revendication 1, comprenant la cyclosporine A [D-2-propylthio-sar3] comme principe actif.

4. Agent de favorisation de la croissance capillaire tel que défini dans la revendication 1, comprenant la cyclosporine A [D-2-isopropylthio-sar3] comme principe actif.

5. Agent de favorisation de la croissance capillaire tel que défini dans la revendication 1, comprenant la cyclosporine A [D-2-allylthio-sar3] comme principe actif.

6. Agent de favorisation de la croissance capillaire tel que défini dans la revendication 1, comprenant la cyclosporine A [D-2-benzylthio-sar3] comme principe actif.

7. Agent de favorisation de la croissance capillaire tel que défini dans la revendication 1, comprenant la cyclosporine A [D-2-(4-nitrophényl)thio-sar3] comme principe actif.

8. Agent de favorisation de la croissance capillaire tel que défini dans la revendication 1, comprenant la cyclosporine A [D-2-(diméthylthiocarbamyl)dithio-sar3] comme principe actif.

9. Agent de favorisation de la croissance capillaire tel que défini dans n'importe laquelle des revendications 1 à 8, lequel est formulé sous une forme sélectionnée dans le groupe consistant en formulation liquide, spray, gel, pâte, émulsion, crème, après-shampooing et shampooing.
